Europäisches Patentamt

European Patent Office

Office européen des brevets

(11) Publication number: **0 353 267 B1**

(12) EUROPEAN PATENT SPECIFICATION

(45) Date of publication of patent specification: **13.10.93**  (51) Int. Cl.⁵: **A61K 31/23**, A61K 9/107, A61K 49/04

(21) Application number: **89900673.8**

(22) Date of filing: **14.12.88**

(86) International application number:
**PCT/SE88/00680**

(87) International publication number:
**WO 89/05638 (29.06.89 89/14)**

Consolidated with 88850423.0/0321429
(European application No./publication No.) by
decision dated 17.10.91.

The file contains technical information submitted
after the application was filed and not included in
this specification

(54) EMULSION FOR PARENTERAL ADMINISTRATION.

(30) Priority: **18.12.87 SE 8705064**

(43) Date of publication of application:
**07.02.90 Bulletin 90/06**

(45) Publication of the grant of the patent:
**13.10.93 Bulletin 93/41**

(84) Designated Contracting States:
**AT BE CH DE ES FR GB GR IT LI LU NL SE**

(56) References cited:
**EP-A- 145 873**
**DE-A- 3 409 793**
**FR-A- 2 553 661**
**US-A- 3 158 541**
**US-A- 3 198 704**

(73) Proprietor: **Kabi Pharmacia AB**
**Rapsgatan 7**
**S-751 82 Uppsala(SE)**

(72) Inventor: **WRETLIND, Karl, Arvid, Johannes**
**Floragatan 2**
**S-114 31 Stockholm(SE)**
Inventor: **AJAXON, Bengt, Magnus**
**Hävelvägen 7**
**S-756 47 Uppsala(SE)**

(74) Representative: **Onn, Thorsten et al**
**AB Stockholms Patentbyra**
**Zacco & Bruhn**
**P.O. Box 23101**
**S-104 35 Stockholm (SE)**

Patent Abstracts of Japan, vol. 10, no. 107
(C-341)

**Description**

Fat emulsions which are intended, inter alia, for intravenous, nutrient supply and which exhibit insignificant secondary effects have been available since the beginning of the 1960's (Wretlind, A. Development of fat emulsions, JPEN 5: No. 3, 230.235, 1981). This development work has investigated the effect of emulsions which contain a number of mutually different fats (olive oil, cottonseed oil, soybean oil, maize oil, safflower oil, coconut oil, etc.) and several mutually different emulsifiers (soybean phospholipids, egg yolk phospholipids, cerebrosides, diglycerides etc.). One characteristic feature common to all of these emulsions is that the fats or oils used comprise triglycerides of fatty acids.

All of the fat emulsions earlier investigated and present day fat emulsions require preparation by homogenization under high pressure. One reason for this is because the fats used have high viscosities. Another drawback with present day fat emulsions is that the fats or oils used can only be cleansed with great difficulty, in a manner to free the glycerides completely from all other substances, such as sterols and unsaponifiable fractions. Several methods have been proposed for cleansing the oils used. One such method has been described by S.S. Chang (US-A 4 101 673). The method to Chang involves removing a part of the polar, undesirable constituents with the aid of silica gel. Other attempts have been made with the aid of molecular distillation techniques. This latter method has not been found to have any practical value, however.

Consequently, there is a desire in this respect to find compounds other than triglycerides which will enable fatty acids to be administered in the form of emulsions, and optionally in the form of emulsions which are totally free from the so-called unsaponifiable residues present in fats of animal and vegetable origins. Another desire is one of discovering fatty acid compounds which have a lower viscosity than the lipids used hitherto and at present in the preparation of fat emulsions. A lower viscosity would also enable fat emulsions to be prepared in a somewhat simpler fashion, and would also enable emulsions to be prepared with smaller particle sizes of the colloidal suspension than those of conventionally prepared fat emulsions.

The present invention now makes it possible to prepare emulsions which will satisfy the aforesaid desiderata, with the use of alkyl esters, and then preferably ethyl esters of fatty acids deriving from synthetic, animal or vegetable origins. These alkyl esters can be obtained either by esterification of triglycerides with ethyl alcohol or other alcohols in the presence of a catalyst, such as sodium alcoholate and certain zinc compounds. Alternatively, fatty acids can be prepared by complete hydrolysis of triglycerides with sodium hydroxide or potassium hydroxide, whereafter the solution containing the salt compound of the fatty acids is extracted with hexane or some other organic solvent, so as to remove unsaponifiable residues. The free fatty acids are obtained subsequently to adding hydrochloric acid or some other acid. The free fatty acids can then be converted to ethyl esters or some other alkyl esters in some suitable way, by treating the fatty acids with ethyl alcohol or some other alkyl alcohol having one or two hydroxyl groups capable of esterification. Suitable esters can also be obtained by esterifying synthetic or otherwise produced fatty acids having an even or an odd number of carbon atoms.

The present invention thus relates to an emulsion intended for parenteral administration and including a hydrophobic phase emulsified in a water phase, this emulsion being characterized in that a substantial part of the hydrophobic phase comprises one or more alkyl esters of pharmacologically acceptable fatty acids. The alkyl esters will preferably comprise low-molecular alkyl esters having 2-4 carbon atoms in the alkyl group, and then primarily ethyl esters.

The fatty acids are preferably of vegetable, animal or synthetic origins, and will preferably have from 9 to 22 carbon atoms in their carbon chains.

In accordance with one embodiment, the carbon chain of the fatty acids will contain predominantly or exclusively an even or odd number of carbon atoms. This has been found significant with respect to certain usages of the emulsions prepared. For instance, fatty acids having an odd number of carbon atoms will produce a high percentage of glucose when metabolized, a fact which can have significance when using the emulsion as a nutrient source.

The alkyl ester content of the emulsion will suitably be from 5 to 60 percent by weight, calculated on the total emulsion, and preferably from 5 to 30 percent by weight. Future reference to percentages made in this description and appended claims refers to weight/volume percent (w/v), unless otherwise stated.

The hydrophobic phase of the emulsion may also include glyceryl esters of fatty acids. The weight ratio of the alkyl esters to the glyceryl esters will then suitably be from 10:1 to 1:10.

The use of ethyl esters or other alkyl esters of fatty acids will afford, inter alia, the following advantages:

1:o homogenization is simplified as a result of the lower viscosity:

2:o the lower viscosity will also result in a lower viscosity of the prepared emulsion;

3:o a lower intrinsic weight, thereby enabling a lower specific weight to be obtained with, e.g., iodo-emulsions or hydrofluorocarbon emulsions;

4:o ethyl ester results in metabolic properties other than those obtained with triglyceride ester.

It has also been surprisingly found in the case of many substances that, in addition to the aforementioned advantages, these esters are better solvents than triglycerides of animal or vegetable origin. The technique of dissolving pharmacologically active substances in triglycerides, such as soybean oil, and subsequently preparing the solution to emulsion form with the aid of suitable emulsifiers is known to the art, (for instance from US-A 4 168 308). It has been found, however, that in many cases the solubility of these substances in such oils is so poor that desired concentrations in the final emulsion cannot be reached. It has now surprisingly been found that the solvent properties of these alkyl esters, for instance ethyl esters of fatty acids, are totally different from the solvent properties of the triglyceride esters present in the fatty acids of animal and vegetable origins. The use, for instance, of fatty acid ethyl esters enables oil-in-water emulsions to be prepared which contain higher quantities of pharmacologically active substance in the hydrophobic phase. The particle size of these emulsions will also be smaller than the particle size of the emulsions based on triglycerides. Since the particles in an alkyl-ester emulsion are much smaller than the particle size of conventional fat emulsions, there is obtained a much greater specific diffusion surface area, thereby resulting in a more rapid and more powerful effect of the active substance.

According to one important embodiment of the invention, the present emulsion will thus contain one or more pharmacologically active substances dissolved or dispersed in the hydrophobic phase. These substances, or agents, may be of very different types, as will be made apparent in the following.

Furthermore, the inventive emulsion may contain X-ray contrast agents, particularly in the form of one or more iodized fats or contrast substances for such investigative procedures as datortomography and NMRI (Nuclear Magnetic Resonance Imaging). These substances or agents may be present in a quantity of 1-60 percent by weight, calculated on the whole emulsion.

It will also be apparent that the inventive emulsion has two essential areas of use. The first of these areas is the use of the emulsion as a nutrient source intended for parenteral nutrient supply. The second of said areas is the use of the emulsion as a vehicle for carrying pharmacologically active substances, including X-ray contrast substances or media, these substances being dissolved or dispersed in the hydrophobic phase. The afore-illustrated advantages are thus achieved by the superior solvent properties and solution promoting properties of the ingoing alkyl esters. It will be understood, however, that the two areas of use may also be combined, such that an emulsion intended for nutrient supply can include one or more pharmacologically active substances in the hydrophobic phase.

The inventive emulsion is primarily intended for intravenous administration, particularly when used for nutrient supply. The emulsion, however, can also be administered parenterally in any other manner, the manner in which the emulsions are administered being determined by the effect and function of the pharmacologically active substances included and by the indications or symptoms of the patient.

The compositions prepared in accordance with the invention may also contain various additives, i.e. in addition to the active substance (or substances) and the hydrophilic component, comprising water, optionally with substances dissolved therein, and the hydrophobic component, comprising alkyl esters, and then particularly ethyl esters of fatty acids and optionally glyceryl esters of fatty acids. These further additives may, for instances, comprise preserving agents, pH-adjusters and agents for achieving a suitable osmotic pressure. In this respect, one of the most important additives will comprise one or more suitable emulsifiers capable of providing a stable dispersion. A multiple of emulsifying and suspending agents of both natural and synthetic origin can be used in this respect. Examples of such agents include phospholipids deriving from eggs or soybeans, and polyethylene polypropylene glycol. Many useable emulsifiers are known from the literature and are commercially available, and the person skilled in this particular art will have no problem in selecting one or more agents suitable for the purpose intended.

The emulsion may also contain nutrients dissolved or dispersed in the aqueous phase. Examples of such substances include, for instance, amino acids, glycerol, glucose, fructose, xylitol, sorbitol or other sugars or alcohols, water-soluble vitamins, salts and trace elements. The emulsion may contain several of these substances at one and the same time. Furthermore, the aqueous phase may also contain water-soluble, pharmacologically active substances.

All of the particles present in an inventive emulsion will have a diameter considerably smaller than 1 micron, thereby obviating the risk of the particles fastening in the capillaries. An emulsion having a particle size of 0.1-0.3 micron can be produced without any great difficulty. This renders the system stable. It has also been found that the inventive emulsion can be prepared in a manner to prevent the particles from forming agglomerates in the blood. The inventive emulsion will withstand being auto-claved and can be stored for long periods without degradation or decomposition. Furthermore, it has been found that the actual vehicle system is well tolerated and will not result in secondary effects, when administered intravenously.

An example of pharmacologically active substances capable of being administered in accordance with the present invention include those which belong to one of the following groups:

Centrally active substances:

such as    - active depressants
            - anaesthetics
            - active analgesics
            - central stimulants

Substances having peripheral effect on the neuromuscular system:

such as    - spasmolytics
            - muscle relaxing substances

Substances which affect the cardiac and vascular system :

such as    - substances having a vasopressor effect

Substances which affect the respiratory system:

such as    - asthma treating substances

Contrast substances for use in conventional radiology diagnosis, datortomographic and NMRI (Nuclear Magnetic Resonance Imaging) investigations.

Antibiotic, cytostatic and chemotherapeutical substances.

Emulsions prepared in accordance with the present invention exhibit a high degree of tolerance in experiments carried out on animals. Ethyl esters of fatty acids obtained from soybean oil have been examined in infusion experiments on rats. In this respect it was possible to administer 70 ml/kg intravenously at a rate of 0.3 ml/kg/min without the occurrence of secondary effects. The volume used corresponded approximately to the energy consumed each minute by the animal concerned. Subsequent to hydrolysis of the ethyl esters, the amount of alcohol administered to the animals in the aforesaid experiments corresponds to only 10 % of the total energy requirement of the body. This supply of alcohol has no appreciable physiological or medicinal significance. In other experiments, rats were given repeated daily infusions of the aforesaid 10%-ethyl-ester emulsion in an amount of 150 ml/kg over a planned trial period of 14 days. No secondary effects were observed. The animals exhibited a normal weight increase. The amount administered exceeded 40% of the energy requirement of the animals.

The emulsion itself is prepared in a conventional manner, i.e. a manner well known to the skilled person from, for instance, the aforecited literature. Thus, the hydrophobic phase, the emulsifier and the aqueous phase can be mixed together to form a "coarse emulsion", which is then homogenized in some suitable apparatus to a suitable particle size with regard to the hydrophobic phase. Those substances intended to be in solution or dispersion in the hydrophobic phase, and/or the aqueous phase are normally first dissolved in respective phases prior to mixing said phases together. Subsequent to homogenization, the emulsion is poured into suitable containers and then sterilized.

As will be understood, it is imperative that the quality of the emulsion ingredients is such as to be free from pharmacological complaint, and that this quality is sustained through the whole of the process of preparation. Thus, the components must be free from contaminants capable of causing harmful secondary effects, such as pyrogens, and must also be protected from the harmful effect, for instance, of oxidation, prior to, during and subsequent to the process of preparation, all of which is well known to the person skilled in the art.

The invention will now be described with reference to a number of examples.

Example 1

100 g of soybean oil were mixed with 1 liter of absolute alcohol. It was found that the oil did not dissolve, but lay in a layer beneath the alcohol. Sodium alcoholate was then added in an amount corresponding to 0.25 g metallic sodium. A clear solution was obtained after 20-30 minutes, subsequent to

EP 0 353 267 B1

trans-esterification of the soybean oil taking place. Three volumes of water were added and the resultant oil layer was then isolated and washed with small volumes of water. The resultant oil comprised the ethyl esters of the fatty acids of the soybean oil.

The viscosity of the ethyl esters is significantly lower than the viscosity of the original soyabean oil. The density is also lower than the density of the corresponding triglyceride.

50 g of the ethyl esters were mixed with 6 g phospholipids, 12.5 g glycerol and water to a volume of 500 ml. 1 M NaOH was added to obtain a pH between 7 and 10.5, where-after the mixture was homogenized in a conventional manner, e.g. in a Moulin-Gaulin homogenizer. The resultant emulsion was heat sterilized at 120°C for 20 minutes. Subsequent to being analyzed for control purposes, the emulsion was ready for intravenous administration. The measured particle size was 0.15-0.30 micron.

The emulsion was administered in quantities of 150 ml per kilogram of body weight and day to rats under a planned 14 day course of administration. The rats exhibited a good increase in weight. No signs of secondary effects were observed. The amount administered corresponded to about 40 to 50 % of the energy requirements of the rats.

Example 2

100 g of oil (soybean oil, safflower oil, olive oil or some other vegetable or animal oil) were mixed with 2 liters of 0.2 M NaOH and 0.5 liter of hexane while slowly stirring the mixture. The hexane fraction was separated, subsequent to all fat having been saponified. The aqueous solution was neutralized with 1 liter of 0.5 M HCl. The resultant layer of free fatty acids was separated and washed with water. Subsequent to having removed all water with water-free sodium sulphate, the fatty acids were esterified with ethyl alcohol or some other alkyl alcohol in a manner similar to that described, for instance, by C.H. Rogers (A method for manufacturing oenanthylate. J. Amer. Pharmaceut. Assoc. Sci. Ed. Vol 12:503-506, No. 6, 1923.)

The esters obtained were used to prepare fat emulsion. The ingredients used were as follows:

| Ethyl esters of fatty acids | 100 g |
|---|---|
| Egg yolk phospholipids | 12 g |
| Glycerol | 25 g |

Sterile and pyrogen-free water to an amount of 1000 ml Sodium hydroxide solution 1 M in an amount sufficient to obtain a pH of 7-10.5

The ingredients were mixed in a Turmix, Turrax or a similar mixer. The resultant "coarse emulsion" was homogenized in an homogenizer of the type Moulin-Gaulin microfluidizer or the like. The emulsion obtained was sterilized in an autoclave at 120°C for 20 minutes.

Example 3

Ethyl esters of fatty acids obtained from animal or vegetable fat were mixed with phospholipids from egg or soyabean oil and glycerol in the following proportions:

| Ethyl esters of fatty acids | 100 g |
|---|---|
| Phospholipids | 12 g |
| Glycerol | 22.5 g |

These ingredients were thoroughly mixed in a Turmix or Turrax apparatus or similar mixers. Sterile and pyrogen-free water was then added to the mixture to a total volume of 1000 ml. The emulsion obtained will be sterile, provided that the emulsion is prepared from sterile and pyrogen-free ingredients under aseptic conditions. When this is not the case, the emulsion can be heat sterilized. This methodology will provide an emulsion of desirable particle size.

Example 4

Diazepam was dissolved in ethyl esters of fatty acids obtained from animal or vegetable fat, and an emulsion was prepared from the solution. The ingredients were used in the following proportions:

6

**EP 0 353 267 B1**

| Diazepam | 0.5 g |
|---|---|
| Ethyl esters | 10 g |
| Phospholipids from egg | 1.2 g |
| Glycerol | 2.25 g |
| Sterile and pyrogen-free water to | 100 ml |
| Sodium hydroxide solution 1 M to pH | 7-10.5 |

The emulsion was poured into bottles of desired volume and then heat sterilized at 120°C.

The diazepam/ethyl-ester emulsion of this example was compared with a diazepam/soybean emulsion with regard to creaming in vitro with plasma and serum derived from seriously ill patients under intensive care. The method by which creaming is determined is given in Swedish Patent Application No. 8505047-4 filed 25 October 1985. The results obtained are set forth in Tables 1 and 2 below. It will be seen from the results that the creaming activity decreased dramatically (i.e. the creaming time had increased) when ethyl ester was used as the hydrophobic phase in diazepam-containing emulsions, instead of soybean-oil.

Table 1.

Investigations concerning the creaming of diazepam/ethyl-ester emulsion and diazepam/soybean-oil emulsion.

The tests were carried out with serum derived from patients under intensive care.

| Patient | Creaming times (hours) of diazepam emulsions containing: | |
|---|---|---|
| | Soybean-oil | Ethyl ester |
| 497 S-G | 1/2 | 7 |
| 499 H | 1/2 | > 24 |
| 486 H | 1/2 | > 24 |
| 501 H | 1 | > 24 |
| 505 S-G | 1 | > 24 |
| 498 H | 2 | > 24 |
| 499 H | 2 | > 24 |
| 500 H | 2 | > 24 |
| 502 S-G | 2 | 24 |
| 506 S-G | 2 | 24 |
| 488 K-G | 3 | > 24 |
| 495 S-G | 3 | > 24 |
| 503 S-G | 3 | > 24 |
| 507 S-G | 3 | > 24 |
| 494 S-G | 4 | > 24 |
| 496 S-G | 4 | > 24 |

Table 2

Investigations concerning the creaming of diazepam/ethyl ester emulsion and diazepam/soybean-oil emulsion

The tests were carried out with plasma derived from patients under intensive care.

| Patient | Creaming time (hours) of diazepam emulsion, the hydrophobic part of which comprised: | |
| | Soybean-oil | Ethyl ester |
| --- | --- | --- |
| 497 S-G | 1/2 | 4 |
| 506 S-G | 1/2 | 7 |
| 486 H | 1/2 | > 24 |
| 499 H | 1/2 | > 24 |
| 500 H | 1/2 | > 24 |
| 507 S-G | 1 | 24 |
| 502 S-G | 1 | 24 |
| 501 H | 1 | > 24 |
| 505 S-G | 1 | > 24 |
| 488 K-S | 2 | > 24 |
| 494 S-G | 2 | > 24 |
| 495 S-G | 2 | > 24 |
| 498 H | 2 | > 24 |
| 496 S-G | 3 | > 24 |
| 503 S-G | 3 | > 24 |
| 490 K-S | 5 | > 24 |
| 487 K-S | 5 | > 24 |
| 491 S-G | 6 | > 24 |

Example 5

An emulsion was prepared from the following ingredients:

| | |
| --- | --- |
| Pregnenolone | 600 mg |
| Ethyl esters of fatty acids obtained from animal or vegetable fat | 20 g |
| Phospholipids from egg | 1.2 g |
| Glycerol | 2.5 g |
| Sterile and pyrogen-free water to a quantity of | 100 ml |
| Sodium hydroxide solution 1 M to pH | 7-10.5 |

Homogenization was effected in the same manner as that described in Example 2. The emulsion was poured into bottles and heat sterilised at 120°C for 20 minutes.

9

Example 6

| Iodized soybean oil | 30 ml |
| Ethyl esters of fatty acids obtained from soyabean oil | 10 ml |
| Phenylalanine | 0.2 g |
| Phospholipids from egg | 2.0 g |
| Glycerol | 2.25 g |
| Sterile and pyrogen-free water to an amount of | 100 ml |
| Sodium hydroxide solution 1 M to pH | 7.5-10 |

The mixture was homogenized in the same manner as that described in Example 2 and the emulsion was poured into bottles and then heat sterilized at 120°C for 20 minutes.

The particle size of the emulsion was determined in a conventional manner was and found to be between 0.15-0.20 micron.

Example 7

Amphotericin B is an antifungal antibiotic. The substance is soluble in dimethyl acetamide and dimethylsulfoxide, but very difficult to dissolve in water and common organic solvents. An infusion suspension can be prepared with sodium deoxycholate. This suspension, however, is highly unstable and must therefore be used within 8 hours from the time of its preparation. It has now been found that a stable emulsion having the following composition can be prepared when ethyl esters from vegetable oils are used as a solution promotor.

| Amphotericin B | 75 mg |
| Ethyl esters of fatty acids from soybean oil | 15 g |
| Soybean oil | 22.5 g |
| Phospholipids from egg | 3.37 g |
| Glycerol | 6.75 g |
| Sterile and pyrogen-free water to an amount of | 300 ml |
| Sodium hydroxide solution 1 M to pH | 7.5-10 |

The emulsion was prepared and sterilized in the same way as that described in Example 2.

Example 8

| Perfluorodecalin (Flutec PP5 from ISC Chemicals Ltd.) | 28 g |
| Ethyl esters of fatty acids from soybean oil | 10 g |
| Phospholipids from egg | 1.2 g |
| Glycerol | 2.5 g |
| Sterile and pyrogen-free water to an amount of | 100 ml |
| Sodium hydroxide solution 1 M to pH | 7-10 |

The emulsion was prepared and sterilized in the same manner as that described in Example 2.

**Claims**
**Claims for the following Contracting States : AT, BE, CH, DE, FR, GB, IT, LI, LU, NL, SE**

1. An emulsion for parenteral administration, comprising a hydrophobic phase emulsified in an aqueous phase by means of one or more emulsifying agents, one or more pharmacologically active agents being dissolved or dispersed in the hydrophobic phase, characterized in that the emulsifying agent comprises a phospholipide from eggs or soybeans, the emulsion has a particle size from 0.1 to 0.3 μm, and that the hydrophobic phase contains one or more alkyl esters of pharmacologically acceptable fatty acids having 2 - 4 carbon atoms in the alkyl part in an amount of 5 to 60 percent by weight.

2. An emulsion according to claim 1, characterized in that the alkyl esters are ethyl esters.

3. An emulsion according to claim 1 or 2, characterized in that the hydrophobic phase also includes glyceryl esters of fatty acids, the weight ratio of alkyl esters to glyceryl esters being from 10:1 to 1:10.

4. An emulsion according to any of claims 1-3, characterized in that the aqueous phase of the emulsion contains amino acids and/or glycerol, fructose, xylitol and/or sorbitol.

5. An emulsion according to any of claims 1-4, characterized in that the pharmacologically active substance or substances are selected from the groups of active depressants, anaesthetics, analgesics, central stimulants, spasmolytics, muscle relaxing substances, substances which affect the cardiac and vascular system, such as vasodepressors, substances which affect the respiratory system, such as asthma agents, contrast substances for X-ray or NMRI investigations, antibiotics, cytostatics and chemotherapeutics.

6. An emulsion according to any of claims 1-5, characterized in that the emulsion has dissolved or dispersed in the alkyl esters at least one iodized fat, iodized fatty acid alkyl ester or perfluorcarbon compound in an amount of 1 - 60 percent by weight.

7. An emulsion according to any of claims 1-6, characterized in that the carbon chains of the fatty acid parts in the alkyl esters contain 9-22 carbon atoms.

8. An emulsion according to any of claims 1-7, characterized in that it contains alkyl esters of synthetic fatty acids or fatty acids produced in some other way having an even or an odd number of carbon atoms.

**Claims for the following Contracting States : ES, GR**

1. A method for the preparation of an emulsion for parenteral administration, wherein a hydrophobic phase is emulsified in an aqueous phase by means of one or more emulsifying agents, on or more pharmacologically active agents being dissolved or dispersed in the hydrophobic phase, characterized in that the emulsifying agent comprises a phospholipide from eggs or soybeans, the emulsion is prepared with a particle size from 0.1 to 0.3 $\mu$m, and that as the hydrophobic phase is used one containing one or more alkyl esters of pharmacologically acceptable fatty acids having 2 - 4 carbon atoms in the alkyl part, in an amount of 5 to 60 percent by weight.

2. A method according to claim 1, characterized in that the alkyl esters are ethyl esters.

3. A method according to claim 1 or 2, characterized in that in the hydrophobic phase is also incorporated glyceryl esters of fatty acids, the weight ratio of alkyl esters to glyceryl esters being from 10:1 to 1:10.

4. A method according to any of claims 1-3, characterized in that in the aqueous phase is incorporated amino acids and/or glycerol, fructose, xylitol and/or sorbitol.

5. A method according to any of claims 1-4, characterized in that the pharmacologically active substance or substances are selected from the groups of active depressants, anaesthetics, analgesics, central stimulants, spasmolytics, muscle relaxing substances, substances which affect the cardiac and vascular system, such as vasodepressors, substances which affect the respiratory system, such as asthma agents, contrast substances for X-ray or NMRI investigations, antibiotics, cytostatics or chemotherapeutics.

6. A method according to any of claims 1-5, characterized in that in the alkyl esters is dissolved or dispersed at least one iodized fat, iodized fatty acid alkyl ester or perfluorocarbon compound in an amount of 1 - 60 percent by weight.

7. A method according to any of claims 1-6, characterized in that the carbon chains of the fatty acid parts in the alkyl esters contain 9-22 carbon atoms.

8. A method according to any of claims 1-7, characterized in that in the emulsion are incorporated alkyl esters of synthetic fatty acids or fatty acids produced in some other way and having an even or an odd number of carbon atoms.

**Patentansprüche**
**Patentansprüche für folgende Vertragsstaaten : AT, BE, CH, DE, FR, GB, IT, LI, LU, NL, SE**

1. Emulsion für die parenterale Verabreichung, enthaltend eine in einer wäßrigen Phase mittels eines emulgierenden Mittels oder mehrerer emulgierte hydrophobe Phase und einen in der hydrophoben Phase gelösten oder dispergierten Wirkstoff oder mehrere, dadurch gekennzeichnet, daß das emulgierenden Mittel ein Phospholipid aus Eiern oder Sojabohnen enthält und die Emulsion eine Teilchengröße von 0,1 bis 0,3 $\mu$m aufweist, und daß die hydrophobe Phase einen Alkylester oder mehrere von pharmakologisch verträglichen Fettsäuren mit 2 bis 4 Kohlenstoffatomen in dem Alkylteil in einer Menge von 5 bis 60 Gew.-% enthält.

2. Emulsion nach Anspruch 1, dadurch gekennzeichnet, daß die Alkylester Ethylester sind.

3. Emulsion nach Anspruch 1 oder 2, dadurch gekennzeichnet, daß die hydrophobe Phase weiterhin Glycerylester von Fettsäuren enthält, wobei das Gewichtsverhältnis der Alkylester zu den Glycerylestern von 10:1 bis 1:10 beträgt.

4. Emulsion gemäß einem jeden der Ansprüche 1 bis 3, dadurch gekennzeichnet, daß die wäßrige Phase der Emulsion Aminosäuren und/oder Glycerin, Fructose, Xylitol und/oder Sorbitol enthält.

5. Emulsion gemäß einem jeden der Ansprüche 1 bis 4, dadurch gekennzeichnet, daß die pharmakologisch wirksame Substanz oder wirksamen Substanzen aus der Gruppe der Tranquilizer, Anästhetika, Analgetika, zentralen Stimulantien, Spasmolytika, muskelrelaxierenden Substanzen, Substanzen mit Wirkung auf das kardiale und vaskuläre System, wie Vasodrepressoren, Substanzen mit Wirkung auf das respiratorische System wie Asthmamittel, Kontrastsubstanzen für Röntgen- oder NMR-Imaging-Untersuchungen, Antibiotika, Cytostatika und Chemotherapeutika ausgewählt ist bzw. sind.

6. Emulsion gemäß einem jeden der Ansprüche 1 bis 5, dadurch gekennzeichnet, daß die Emulsion in den Alkylestern mindestens ein jodiertes Fett, einen jodierten Fettsäurealkylester oder eine Perfluorkohlenstoffverbindung in einer Menge von 1 bis 60 Gew.-% gelöst oder dispergiert enthält.

7. Emulsion gemäß einem jeden der Ansprüche 1 bis 6, dadurch gekennzeichnet, daß die Kohlenstoffketten der Fettsäureteile in den Alkylestern 9 bis 22 Kohlenstoffatome enthalten.

8. Emulsion gemäß einem jeden der Ansprüche 1 bis 7, dadurch gekennzeichnet, daß sie Alkylester von synthetischen Fettsäuren oder von auf anderem Wege hergestellte Fettsäuren mit einer geraden oder ungeraden Anzahl von Kohlenstoffatomen enthält.

**Patentansprüche für folgende Vertragsstaaten : ES, GR**

1. Verfahren zur Herstellung einer Emulsion für die parenterale Verabreichung, bei dem eine hydrophobe Phase in einer wäßrigen Phase mittels eines emulgierenden Mittels oder mehrerer emulgiert wird, wobei ein pharmakologisch wirksames Mittel oder mehrere in der hydrophoben Phase gelöst oder dispergiert wird, dadurch gekennzeichnet, daß das emulgierende Mittel ein Phospholipid aus Eiern oder Sojabohnen umfaßt sowie die Emulsion mit einer Teilchengröße von 0,1 bis 0,3 $\mu$m hergestellt wird, und daß eine hydrophobe Phase verwendet wird, die einen Alkylester oder mehrere von pharmakologisch verträglichen Fettsäuren mit 2-4 Kohlenstoffatomen in dem Alkylteil in einer Menge von 5 bis 60 Gew.-% enthält.

2. Verfahren nach Anspruch 1, dadurch gekennzeichnet, daß die Alkylester Ethylester sind.

3. Verfahren nach Anspruch 1 oder 2, dadurch gekennzeichnet, daß der hydrophoben Phase weiterhin Glycerylester von Fettsäuren zugesetzt sind, wobei das Gewichtsverhältnis von Alkylestern zu Glycerylestern im Bereich von 10:1 bis 1:10 liegt.

4. Verfahren gemäß einem jeden der Ansprüche 1 bis 3, dadurch gekennzeichnet, daß der wäßrigen Phase Aminosäuren und/oder Glycerin, Fructose, Xylitol und/oder Sorbitol zugesetzt sind.

5. Verfahren gemäß einem jeden der Ansprüche 1 bis 4, dadurch gekennzeichnet, daß die pharmakologisch wirksame Substanz bzw. Substanzen aus der Gruppe der aktivitätsdämpfenden Mittel, Anästhetika, Analgetika, zentralen Stimulantien, Spasmolytika, muskelrelaxierenden Substanzen, Substanzen mit Wirkung auf das kardiale und vaskuläre System, wie Vasodepressoren, Substanzen mit Wirkung auf das respiratorische System wie Asthmamittel, Kontrastsubstanzen für Röntgen- oder NMR-Imaging-Untersuchungen, Antibiotika, Cytostatika und Chemotherapeutika ausgewählt ist bzw. sind.

6. Verfahren gemäß einem jeden der Ansprüche 1 bis 5, dadurch gekennzeichnet, daß in den Alkylestern mindestens ein jodiertes Fett, ein jodierter Fettsäurealkylester oder eine Perfluorkohlenstoffverbindung in einer Menge von 1 bis 60 Gew.-% gelöst oder dispergiert ist.

7. Verfahren gemäß einem jeden der Ansprüche 1 bis 6, dadurch gekennzeichnet, daß die Kohlenstoffketten der Fettsäureteile in den Alkylestern 9 bis 22 Kohlenstoffatome enthalten.

8. Verfahren gemäß einem jeden der Ansprüche 1 bis 7, dadurch gekennzeichnet, daß der Emulsion Alkylester von synthetischen Fettsäuren oder auf anderen Wegen erzeugten Fettsäuren mit einer geraden oder ungeraden Zahl von Kohlenstoffatomen zugesetzt sind.

**Revendications**
**Revendications pour les Etats contractants suivants : AT, BE, CH, DE, FR, GB, IT, LI, LU, NL, SE**

1. Emulsion pour l'administration parentérale, comprenant une phase hydrophobe émulsifiée dans une phase aqueuse au moyen d'un ou plusieurs agents émulsifiants, un ou plusieurs agents actifs pharmacologiquement étant dissous ou mis en dispersion dans la phase hydrophobe, caractérisée en ce que l'agent émulsifiant comprend un phospholipide à partir d'oeufs ou de graines de soja, l'émulsion ayant une taille particulaire de 0,1 à 0, 3 $\mu$m, et en ce que la phase hydrophobe contient un ou plusieurs esters alcoyliques d'acides gras pharmacologiquement acceptables ayant de 2 - 4 atomes de carbone dans la partie alcoyle, dans une quantité de 5 à 60 % en poids.

2. Emulsion selon la revendication 1, caractérisée en ce que les esters alcoyliques sont des esters éthyliques.

3. Emulsion selon la revendication 1 ou 2, caractérisée en ce que la phase hydrophobe comprend également des esters glycéryliques d'acides gras, le rapport pondéral entre les esters alcoyliques et les esters glycéryliques étant de 10:1 à 1:10.

4. Emulsion selon l'une quelconque des revendications 1-3, caractérisée en ce que la phase aqueuse de l'émulsion contient des acides aminés et/ou du glycérol, du fructose, du xylitol et/ou du sorbitol.

5. Emulsion selon l'une quelconque des revendications 1-4, caractérisée en ce que la substance ou les substances actives pharmacologiquement sont choisies parmi les groupes des agents dépresseurs actifs, des anesthésiants, des analgésiques, des stimulants du système central, des spasmolythiques, des substances de relaxation musculaire, des substances qui affectent le système cardiaque et vasculaire telles que les vasodépresseurs, les substances qui affectent le système respiratoire telles que des agents contre l'asthme, des substances de contraste pour les examens aux rayons X ou de résonance magnétique nucléaire, des antibiotiques, des cytostatiques et substances chimiothérapeutiques.

6. Emulsion selon l'une quelconque des revendications 1-5, caractérisée en ce que l'émulsion a dissous ou dispersé dans les esters alcoyliques au moins une matière grasse iodée, un ester alcoylique des acides gras iodés ou un composé de perfluorocarbone dans une quantité de 1 - 60 % en poids.

**7.** Emulsion selon l'une quelconque des revendications 1-6, caractérisée en ce que les chaînes de carbone des parties des acides gras dans les esters alcoyliques contiennent 9-22 atomes de carbone.

**8.** Emulsion selon l'une quelconque des revendications 1-7, caractérisée en ce qu'elle contient des esters alcoyliques des acides gras synthétiques ou des acides gras produits d'une manière ou d'une autre et ayant un nombre pair ou un nombre impair d'atomes de carbone.

**Revendications pour les Etats contractants suivants : ES, GR**

**1.** Procédé pour la préparation d'une émulsion destinée à l'administration parentérale, dans laquelle une phase hydrophobe est émulsifiée dans une phase aqueuse au moyen d'un ou plusieurs émulsifiants, un ou plusieurs agents actifs pharmacologiquement étant dissous ou mis en dispersion dans la phase hydrophobe, caractérisé en ce que l'agent émulsifiant comprend un phospholipide à partir d'oeufs ou de graines de soja, l'émulsion étant préparée avec une taille particulaire de 0,1 à 0, 3 $\mu$m, et en ce qu'en qualité de phase hydrophobe, on utilise une phase contenant un ou plusieurs esters alcoyliques d'acides gras pharmacologiquement acceptables ayant 2 - 4 atomes de carbone dans la partie alcoyle, dans une quantité de 5 à 60 % en poids.

**2.** Procédé selon la revendication 1, caractérisé en ce que les esters alcoyliques sont des esters éthyliques.

**3.** Procédé selon la revendication 1 ou 2, caractérisé en ce que la phase hydrophobe comprend également des esters glycéryliques d'acides gras, le rapport pondéral entre les esters alcoyliques et les esters glycéryliques étant de 10:1 à 1:10.

**4.** Procédé selon l'une quelconque des revendications 1-3, caractérisé en ce que la phase aqueuse de l'émulsion contient des acides aminés et/ou du glycérol, du fructose, du xylitol et/ou du sorbitol.

**5.** Procédé selon l'une quelconque des revendications 1-4, caractérisé en ce que la substance ou les substances actives pharmacologiquement sont choisies parmi les groupes des agents dépresseurs actifs, des anesthésiants, des analgésiques, des stimulants du système central, des spasmolythiques, des substances de relaxation musculaire, des substances qui affectent le système cardiaque et vasculaire telles que les vasodépresseurs, les substances qui affectent le système respiratoire telles que des agents contre l'asthme, des substances de contraste pour les examens aux rayons X ou de résonance magnétique nucléaire, des antibiotiques, des cytostatiques et des substances chimiothérapeutiques.

**6.** Procédé selon l'une quelconque des revendications 1-5, caractérisé en ce que l'émulsion a dissous ou dispersé dans les esters alcoyliques au moins une matière grasse iodée, un ester alcoylique des acides gras iodés ou un composé de perfluorocarbone dans une quantité de 1 - 60 % en poids.

**7.** Procédé selon l'une quelconque des revendications 1-6, caractérisé en ce que les chaînes de carbone des parties des acides gras dans les esters alcoyliques contiennent 9-22 atomes de carbone.

**8.** Procédé selon l'une quelconque des revendications 1-7, caractérisé en ce que l'émulsion contient des esters alcoyliques des acides gras synthétiques ou des acides gras produits d'une manière ou d'une autre et ayant un nombre pair ou un nombre impair d'atomes de carbone.